# DEMANDE DE BREVET EUROPEEN

(11) **EP 0 680 769 A1**
(43) Date de publication de la demande: **08.11.1995**
(21) Numéro de dépôt: 95401008.8
(22) Date de dépôt: 02.05.1995
(51) Int. Cl.: A61M 16/00

(54) **Ventilateur à usage médical**

(30) Priorité: 05.05.1994 FR 9405511
(71) Demandeur: BIOMS S.A., F-64000 Pau (FR)
(72) Inventeur: Legrand, Michel, F-64230 Lescar (FR)
(74) Mandataire: Epstein, Henri

(57) **Abrégé**

Appareils respirateurs pour ventilation à domicile, dont le principe est basé sur la compression d'un soufflet.

Le moteur pas à pas (5) entraine en mouvement alternatif de va-et-vient deux courroies parallèles (16) auxquelles est fixée par ses deux bouts une barre d'entrainement (15) accouplée à la plaque de base (14) du soufflet (7).

En cad d'arrêt du moteur (5), la barre d'entrainement (15) se trouve propulsée vers le haut sous l'action du ressort en spirale (20) associé à la barre, la plaque de base (14) du soufflet exerce une poussée sur la tige (22) qui fait pivoter le clapet (21) ouvrant le passage d'air. L'emploi du mouvement alternatif du soufflet combiné à l'action du ressort en spirale assure la sécurité d'emploi de l'appareil.

## Description

La présente invention est relative aux ventilateurs à usage médical, tels qu'appareils respirateurs pour ventilation à domicile, dont le principe de la ventilation est basé sur la compression d'un soufflet déplaçant un volume d'air envoyé vers les poumons du patient.

Les appareils connus de ce genre étant souvent encombrants et n'offrant pas toutes les garanties de sécurité d'emploi, ils présentent des inconvénients pour l'usage à domicile par le patient.

La présente invention a pour but de remédier aux inconvénients précités et elle propose un ventilateur à usage médical, tel que respirateur pour ventilation à domicile, dont le module de ventilation comporte un soufflet dont la tête est reliée à une prise d'air ambiant ou enrichi en oxygène et à un tuyau d'alimentation du patient, la base du soufflet étant accouplée à un mécanisme d'entrainement lui imprimant un mouvement alternatif de va-et-vient vertical et qui est caractérisé en ce que la base du soufflet est accouplée à une barre horizontale d'entrainement, laquelle est fixée à ses deux bouts aux deux courroies auxquelles un moteur pas à pas imprime un mouvement alternatif de va-et-vient par l'intermédiaire d'un axe de poulies inférieures, lequel axe est associé à un ressort en spirale qui se trouve à l'état contraint lorsque, en état de marche du moteur, la barre d'entrainement se trouve en position basse, tandis que, lors de l'arrêt du moteur, l'énergie de détente dudit ressort en spirale fait remonter la barre d'entrainement à sa position haute d'arrêt, le soufflet étant entièrement replié.

Selon une forme préférée de l'invention, le soufflet est monté sur un plateau de tête dont l'ouverture destinée à la prise d'air communique avec un logement de soupape munie d'un clapet, dont le ressort de rappel assure la fonction anti-retour.

Avantageusement, à proximité du logement de soupape est placée une tige d'ouverture du clapet, dont le poussoir subit l'action de poussée que lui imprime la base inférieure du soufflet lors de sa remontée en position haute sous l'action du ressort en spirale, dont la détente met en rotation l'axe de poulies d'entrainement des courroies.

Le degré de compression du soufflet en état de marche de l'appareil peut être commandé par un capteur de position de référence, dont les signaux commandent la marche du moteur pas à pas.

Le capteur utilisé de préférence est un capteur à effet Hall coopérant avec un aimant dont est équipée la barre d'entrainement.

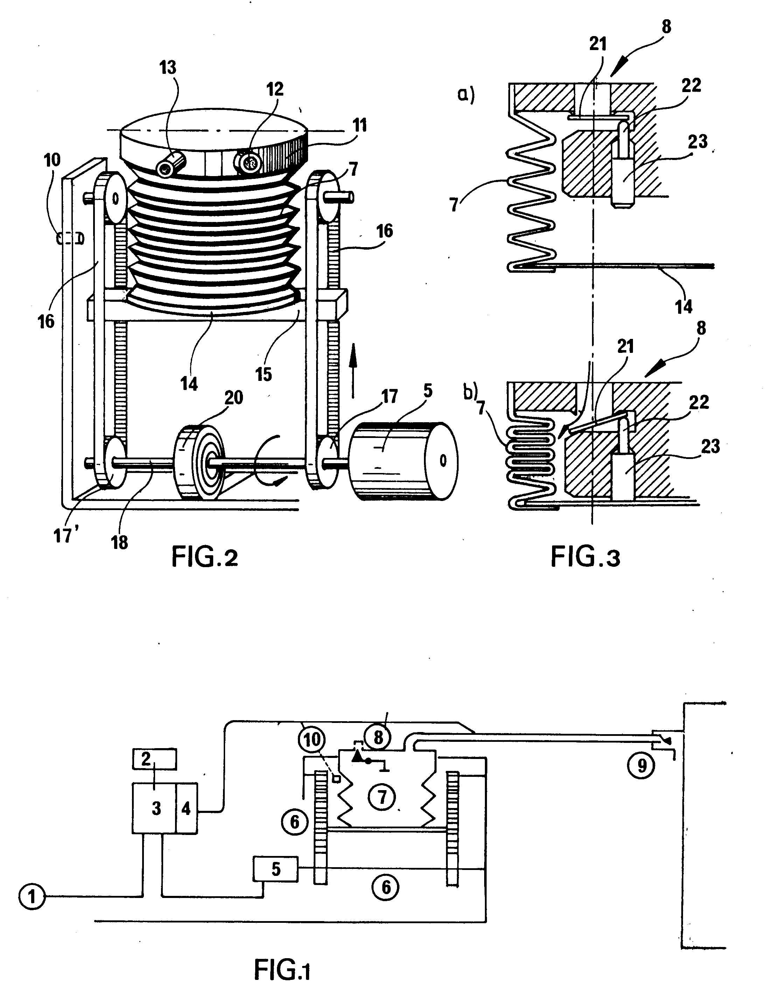

D'autres particularités de l'invention apparaitront à la lumière de la description d'une forme de réalisation de l'appareil selon l'invention illustrée par les dessins, dont
la figure 1 présente un schéma général de l'appareil
la figure 2 montre une vue en perspective du module de ventilation, et
les figures 3a et 3b présentent des vues en coupe montrant respectivement la soupape fermée et ouverte.

L'alimentation électrique 1 fournit le courant à l'ensemble électronique de commande 3 équipé de tableau de réglage 2 et au moteur pas-à-pas 5 entrainant le soufflet 7 du module de ventilation 6. Le patient est relié par un tuyau d'alimentation 9 au module de ventilation 6.

Sur la tête du soufflet 7 est placée une soupape 8 assurant en cas d'arrêt du moteur la mise automatique à l'air libre de la tête du soufflet.

Un capteur de pression 4 permet de régler la pression d'air d'alimentation à sa valeur de consigne.

Un capteur de position de référence 10 sert à assurer la compression du soufflet jusqu'à sa position de référence.

Le module de ventilation 6 comprend un soufflet 7 dont la tête 11 est munie de deux ouvertures, l'une 12 servant d'entrée d'air par l'intermédiaire d'une soupape unidirectionnelle 8 et l'autre 13 destinée à être reliée au tube d'alimentation du patient 9.

La plaque de base 14 du soufflet 7 est accouplée à une barre d'entrainement 15 dont les deux extrémités sont fixées aux deux courroies crantées 16. Les courroies 16 sont entrainées en mouvement par une poulie motrice 17 fixée sur le même axe 18 que la poulie de guidage 17' de l'autre courroie 16 actionnée dans son mouvement alternatif de va-et-vient par un moteur pas à pas 5.

L'axe 18 est associée à un ressort en spirale 20 destiné à emmagasiner l'énergie lorsque l'action du moteur provoque la descente de la barre 15 et à rendre l'énergie emmagasinée lors de la remontée de la barre 15.

Le logement de la soupape 8 placée dans la tête du soufflet 11 contient un clapet 21 et son ressort de rappel non représenté, ainsi qu'une tige 22 coulissante, dont l'extrémité supérieure peut prendre appui sur une extrémité du clapet 21, lorsque le poussoir 23 solidaire de la tige est sollicité par une poussée verticale.

Le fonctionnement du module de ventilation est le suivant.

Lors de la mise en marche, le moteur pas à pas 5 entraine en mouvement alternatif de va-et-vient les deux courroies 16 qui à leur tour accompagnent le mouvement de la barre d'entrainement 15 accouplée à la plaque de base 14 du soufflet 7.

Lors de son actionnement, le soufflet aspire par la soupape unidirectionnelle 8 l'air ambiant ou enrichi en oxygène et l'envoie vers la tuyauterie du patient (9,13).

Des filtres antibactériens peuvent être placés à l'entrée et à la sortie de l'air entrant et sortant du module de ventilation.

Le capteur de position de référence 10 déplace un volume d'air réglable vers les poumons du patient, grâce au répérage de sa position. Il peut être constitué par un capteur à effet Hall coopérant avec un aimant placé sur la barre d'entrainement.

Le capteur de pression 4 veille à ce que la pression ne dépasse pas une valeur de consigne. Dans le cas contraire, le moteur arrête d'actionner le soufflet.

En cas d'arrêt du moteur, la barre d'entrainement 15 se trouve propulsée vers le haut sous l'action du ressort en spirale 20 et, lorsqu'elle est dans sa position extrême, la plaque de base 14 du soufflet exerce une poussée sur le poussoir 23, dont la tige 22 fait pivoter le clapet 21 qui livre le passage d'air vers la tête du soufflet et vers l'utilisateur.

L'emploi du mouvement alternatif du soufflet combiné à la mesure de sécurité due à l'action du ressort en spirale en cas d'arrêt inopiné du moteur apporte des avantages appréciables, tels que la fermeture du soufflet à l'arrêt pour assurer la respiration libre, ainsi qu'une économie d'énergie du moteur, l'énergie du ressort en spirale participant à l'effort de remontée du soufflet.

Le répérage de positionnement grâce au capteur et le mode d'actionnement par moteur pas à pas permettent au système de régulation électronique de placer le soufflet avec précision à la position requise par la sélection des paramètres de ventilation.

## Revendications

1. Ventilateur à usage médical, tel que respirateur pour ventilation à domicile, dont le module de ventilation (6) comporte un soufflet (7) dont la tête (11) est reliée à une prise d'air ambiant (12) ou enrichi en oxygène et à un tuyau d'alimentation du patient (9,13), la base du soufflet (14) étant accouplée à un mécanisme d'entrainement lui imprimant un mouvement alternatif de va-et-vient vertical, caractérisé en ce que la base du soufflet (14) est accouplée à une barre d'entrainement (15), laquelle est fixée à ses deux bouts aux deux courroies (16) auxquelles un moteur pas à pas (5) imprime un mouvement alternatif de va-et-vient par l'intermédiaire d'un axe (18) de poulies inférieures (17,17'), lequel axe est associé à un ressort en spirale (20) qui se trouve à l'état contraint, lorsque, en état de marche du moteur, la barre d'entrainement (15) se trouve en position basse, tandis que, lors de l'arrêt du moteur (5), l'énergie de détente dudit ressort en spirale (20) fait remonter la barre d'entrainement (15) à sa position haute d'arrêt, le soufflet (7) étant entièrement replié.

2. Ventilateur selon la revendication 1, caractérisé en ce que le soufflet (7) est monté sur un plateau de tête (11) dont l'ouverture destinée à la prise d'air (12) communique avec un logement de soupape (8) munie d'un clapet (21), dont un ressort de rappel assure la fonction anti-retour.

3. Ventilateur selon la revendication 1 ou 2, caractérisé en ce qu'à proximité du logement de soupape (8) est placée une tige (22) d'ouverture du clapet, dont le poussoir (23) subit l'action de poussée que lui imprime la base inférieure du soufflet (14) lors de sa remontée en position haute sous l'action du ressort en spirale, dont la détente met en rotation l'axe (18) de poulies d'entrainement des courroies.

4. Ventilateur selon la revendication 1, caractérisé en ce que, en état de marche de l'appareil, le degré de la compression du soufflet est commandé par un capteur de position de référence (10), dont les signaux électriques commandent la marche du moteur pas à pas (5).

5. Ventilateur selon la revendication 4, caractérisé en ce que.le capteur (10)est un capteur à effet Hall coopérant avec un aimant dont est équipée la barre d'entrainement (15).
